# FASCICULE DE BREVET EUROPEEN

(11) **EP 0 780 096 B1**
(45) Date de publication et mention de la délivrance du brevet: **24.05.2000**
(21) Numéro de dépôt: 96402841.9
(22) Date de dépôt: 20.12.1996
(51) Int. Cl.: A61B 17/82, A61B 17/58

(54) **Lien de cerclage à usage médical et matériel de pose**
Umreifungsband für medizinische Zwecke und Einbaugerät
Binding strap for medical uses and fixing device

(30) Priorité: 22.12.1995 FR 9515793
(43) Date de publication de la demande: 25.06.1997
(73) Titulaire: Transystème S.A., 30000 Nimes (FR)
(72) Inventeur: Boutet, Robert-Louis, 34070 Montpellier (FR)
(74) Mandataire: Ravina, Bernard

(56) Documents cités:
- CH-A- 395 666
- US-A- 4 015 311
- US-A- 4 667 662
- US-A- 4 813 416
- US-A- 5 366 461

## Description

La présente invention a pour objet un lien de cerclage à usage médical et matériel de pose.
De tels liens bio-compatibles qualifiés d'implants chirurgicaux humains sont connus.
Ils sont utilisables pour la réduction et le maintien de toute disjonction osseuse ou cartilagineuse.
Ce sont des outils d'ostéosynthèse à usage général par exemple :
- en chirurgie cardiaque pour réaliser une réduction sternale ;
- en chirurgie pulmonaire pour réaliser des réductions costales ;
- en chirurgie orthopédique pour réaliser des réductions femorales, tibiales, d'épaule, de rachis de bassin ou autres.

De tels liens connus sont généralement constitués d'un fil d'acier qui présentent plusieurs inconvénients notamment un phénomène de cisaillement, une impossibilité de contrôle difficile de la tension et l'impossibilité de permettre un verrouillage provisoire offrant l'avantage de pouvoir ouvrir à nouveau et refermer.

Le brevet US BLASNIK n° 5,366,461 décrit un lien avec une aiguille à une extrémité et une chape à l'autre extrémité.
Le lien présente des perforations dans l'une desquelles s'engage une dent de la chape lors du serrage en empêchant tout déserrage par retour en arrière du lien.
Cette disposition interdit un verrouillage provisoire.

Le brevet US POLLACK n° 4,813,416 décrit un lien du même type que le précédent avec perforations et dents de blocage.

Dans le cas de ces deux brevets, le verrouillage est irréversible.

De plus, dans ces brevets, la chape n'est pas de forme tubulaire, cette forme contribuant à la fixation du lien et à son blocage.

La présente invention vise à obvier à ces inconvénients.

Cet objectif est résolu par les caractéristiques de l'invention.

L'utilisation d'un feuillard au lieu et place d'un fil évite les effets de cisaillement.

Suivant une autre caractéristique de l'invention, le feuillard et la chape sont réalisés en acier inoxydable.

Suivant encore une autre caractéristique de l'invention, la chape a une forme tubulaire de section carrée surmontée des oreilles.

Suivant encore une autre caractéristique de l'invention, le feuillard est fixé à la chape par enroulement autour de la paroi inférieure de la chape, cet enroulement laissant le passage au brin libre du feuillard après enlèvement de l'aiguille de mise en place, ledit brin libre étant ensuite replié sur le dessus de la chape entre les oreilles qui sont rabattues pour la fixation définitive du feuillard.
Ce lien présente l'avantage d'une grande surface d'appui avec une grande résistance en réalisant des réductions avec un serrage adapté à tous les cas de figures d'intervention chirurgicale.

De ce fait, il présente en réduction costale une diminution très importante des douleurs post-opératoires.

Dans le cas de réductions sternales, il élimine le risque de fractures sternales transverses par augmentation de la surface d'appui et donc diminution de la pression exercée.
Dans les cas de réduction de fracture sur prothèse de hanche, il permet une réduction de grande qualité au niveau de la queue de prothèse où les vis corticales ne peuvent être implantées.

D'autres avantages et caractéristiques de l'invention apparaîtront à la lecture de la description ci-après de l'invention représentée à titre d'exemple non limitatif aux dessins annexés dans lesquels :
- la figure 1 représente le lien selon l'invention avant usage,
- la figure 2 représente le lien selon l'invention en cours de pose,
- la figure 3 représente en vue partielle la chape et le lien,
- la figure 4 représente un cas d'utilisation du lien en réduction sternale,
- les figures 5, 6, 7 et 8 représentent l'outillage ancillaire de pose.

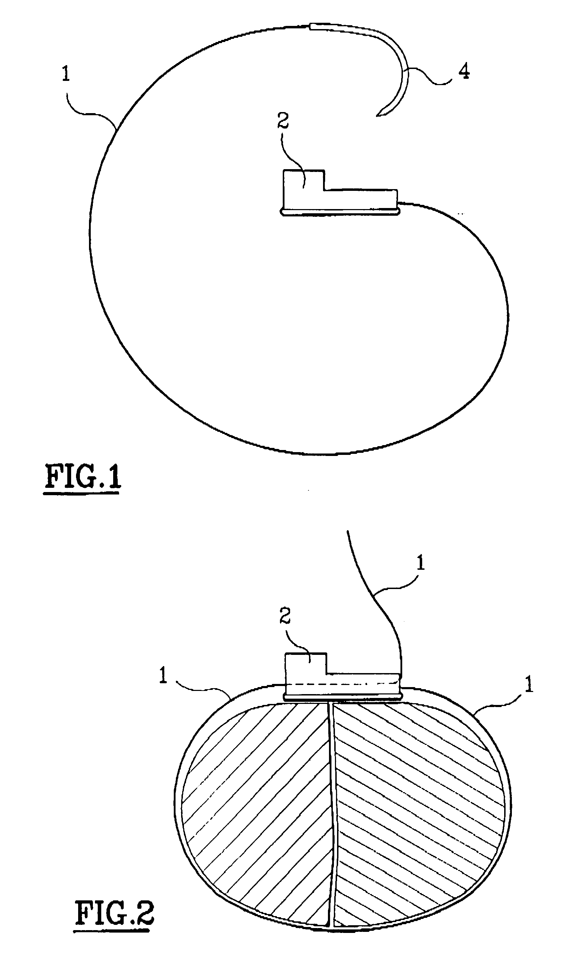

Tel que représenté, le lien selon l'invention (figure 1) comprend un feuillard 1 plat solidaire d'une chape tubulaire 2 à oreilles 3 repliables.
Ledit feuillard est fixé par une extrémité à la chape et est pourvu à son autre extrémité d'une aiguille chirurgicale 4. L'ensemble feuillard 1 et chape 2 est de préférence réalisé en un ou des matériaux bio-compatibles adaptés par exemple en acier inoxydable.

La chape a une forme tubulaire aplatie de section carrée ou rectangle de faible épaisseur présentant une paroi inférieure 5 et une paroi supérieure 6 séparées par un intervalle.

Une portion de la paroi supérieure 6 est découpée pour former les oreilles 3 dressées verticalement et qui sont repliables pour parachever le cerclage comme expliqué plus loin.

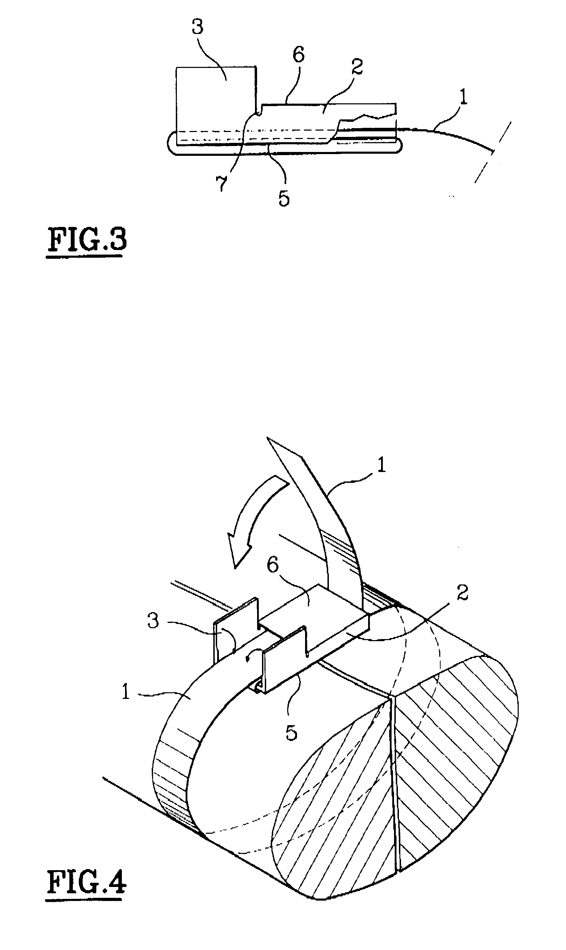

La paroi supérieure 6 est plus courte que la paroi inférieure 5.
Les oreilles 3 sont positionnées à l'opposé du développement du feuillard lors de son enroulement autour de la pièce anatomique à cercler et de préférence en arrière de la chape.
De préférence, une rainure 7 ou sillon est ménagé sur chaque paroi latérale de la chape dans le prolongement de chaque oreille 3 (figure 3).

En figure 3 est représentée la solidarisation de la chape 2 et du feuillard 1.
Cette solidarisation se fait par enroulement du feuillard 1 autour de la paroi inférieure 5 de la chape.
A cet effet, au moins un brin du feuillard sort de la chape, est enroulé sous sa paroi inférieure 5 et retraverse la chape.

De préférence, en fonction de la résistance à la traction souhaitée, il est réalisé un double enroulement du feuillard sur la partie inférieure de la chape.
Le feuillard peut être relié à la chape par au moins un brin extérieur et deux brins intérieurs ou plusieurs brins intérieurs et extérieurs.

L'enroulement du feuillard dans la partie tubulaire de la chape a une hauteur ou épaisseur inférieure à la hauteur de celle-ci entre ses deux parois 5 et 6, ce qui permet le passage de l'extrémité libre du feuillard entre ces deux parois, après enlèvement de l'aiguille 4 pour réaliser le cerclage provisoire.
Le cerclage définitif est obtenu par repliement du brin libre sur la paroi supérieure 6 de la chape et le rabattement des oreilles 3 sur ledit feuillard.

Lors de cette opération, le brin libre du feuillard qui passe par-dessus la paroi 6 est replié par les oreilles 3 dans la chape, sous le niveau de la paroi 6, ce qui réalise un blocage par obstacle de la paroi 6 et les oreilles 3 en raison du sillon ou rainure 7 viennent se replier au même niveau que la paroi 6 de la chape.
La mise en place du lien s'effectue comme suit :

Après mise en place autour de la partie à cercler, le feuillard est renfilé dans la partie libre de la chape (entre l'enroulement de fixation feuillard-chape et la paroi supérieure 6).
La mise en tension s'effectue par opposition entre la chape et le brin libre du lien.

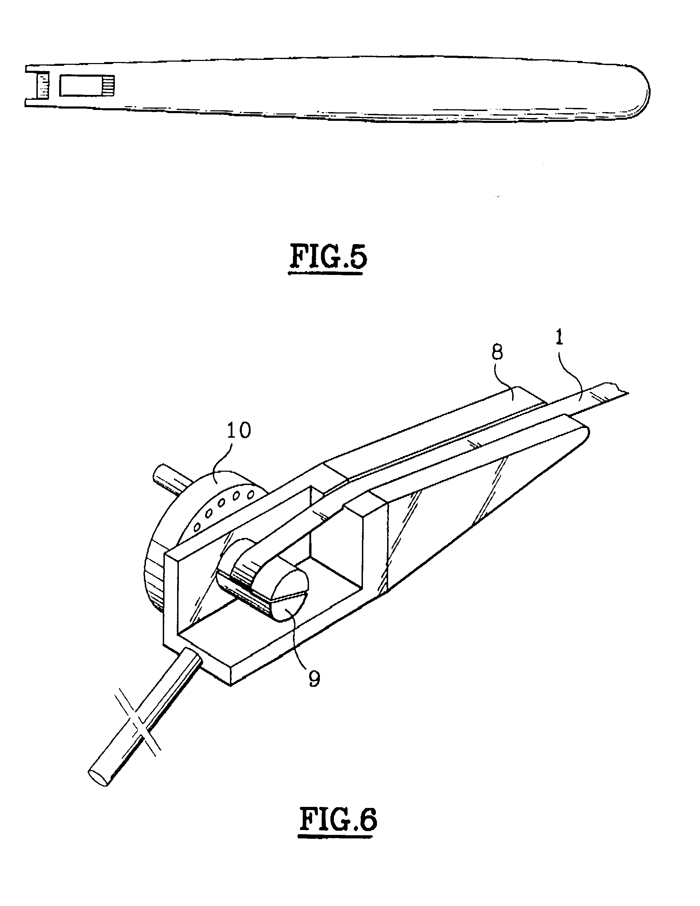

L'ensemble est tenu en position par le repoussoir représenté à la figure 5.
Le repoussoir est constitué d'une lame doté d'une lumière rectangle qui enserre l'ensemble et permet le passage, dans ladite lumière du brin libre.
Le brin libre du feuillard est tendu au moyen d'un porte aiguille.

Lorsque la tension désirée est atteinte, le lien est replié vers la chape, ce qui réalise un verrouillage provisoire.
Lorsque l'ensemble des liens sont verrouillés provisoirement et que la réduction est constatée, chaque lien est positionné entre les oreilles 3 de la chape et celles-ci sont repliées sur le lien afin de la verrouiller définitivement.
Le brin libre est alors coupé au ciseau.

Dans le cas de lien plus large nécessitant un appui plus important, un serrage est effectué au moyen de la pièce de tension à cliquet représentée à la figure 6.
Celle-ci comprend une rampe 8 sur laquelle s'engage le brin libre et un arbre rotatif 9 à fente commandé par une manivelle rotative 10 à cliquet ; une fente de l'arbre reçoit l'extrémité du brin libre pour enroulement, le cliquet permettant de doser l'effort.

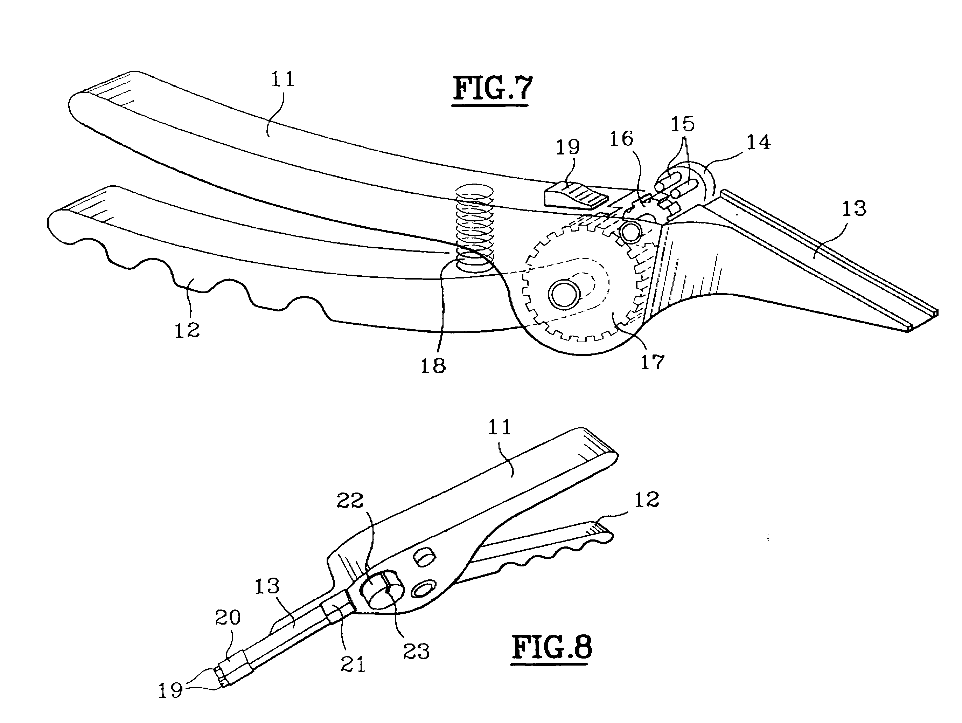

En figure 7 est représentée une pince de tension constituée de deux branches 11-12 articulées.
La branche 11 porte une rampe 13 inclinée. Une roue 14 dotée de pions horizontaux parallèles 15 est entraînée par un pignon cranté 16 coaxial lui-même actionné par une roue dentée 17 portée par la branche 12 articulée à la précédente avec un ressort d'écartement 18 entre elles.
Le brin libre du feuillard est introduit entre les pions.
Un poussoir 19 permet un blocage en position de la pince.

En figure 8 est représentée une autre forme de réalisation de la pince de tension.
Cette pince comporte une rampe 13 déportée latéralement par rapport aux branches 11-12.
L'extrémité de la rampe 13 présente deux nervures saillantes 19 espacées, ménageant un U ouvert.

Sur la rampe 13 est réalisé un premier étrier 20 à proximité de son extrémité inférieure et un deuxième étrier 21 à proximité de son extrémité inférieure.
Dans l'axe de la rampe 13, sur la branche fixe 11, est montée rotative, sous l'action de la poignée 12, une roue 22 avec fente 23.
Pour la mise en tension du lien, le feuillard, après passage dans la chape, est introduit sur la rampe sous les étriers 20-21 et son extrémité est placée dans la fente 23 de la roue 22 qui exerce la traction.

Les liens selon l'invention peuvent être réalisés en plusieurs dimensions et épaisseur en fonction des cas d'utilisation.

## Revendications

1. Lien de cerclage à usage chirurgical comprenant un feuillard (1) plat solidaire à une extrémité de la paroi inférieure (5) d'une chape (3) sur laquelle le brin libre du feuillard après cerclage est replié et bloqué par des oreilles (3) rabattables caractérisé en ce que la chape a une partie tubulaire de section carrée ou rectangle présentant une paroi inférieure (5) et une paroi supérieure (6) par rapport au brin libre dans la chape, que le brin libre est repliable sur la paroi supérieure (6) pour réaliser un cerclage provisoire, que la paroi supérieure (6) est plus courte que la paroi inférieure (5) et que le brin libre du feuillard est repliable dans la chape (2) sous le niveau de la paroi supérieure (6) par repliement des oreilles (3) pour le blocage définitif par obstacle du brin libre du feuillard.

2. Lien de cerclage selon la revendication 1 caractérisé en ce que les oreilles (3) sont séparées de la paroi supérieure (6) de la chape par un sillon (7) de chaque paroi latérale de la chape qui permet leur repliement sous le niveau de la paroi supérieure (6).

3. Lien de cerclage selon la revendication 1 et la revendication 2 caractérisé en ce que les oreilles (3) sont positionnées à l'opposé du développement du feuillard (1) lors de son enroulement autour de la pièce anatomique à cercler et en arrière de la chape.

4. Lien selon la revendication 1 et la revendication 2 caractérisé en ce que le feuillard (1) est enroulé autour de la paroi inférieure (5) de la chape et présente au moins un brin extérieur et deux brins intérieurs.

5. Lien selon la revendication 3 caractérisé en ce que l'enroulement du feuillard (1) présente plusieurs brins intérieurs à la partie tubulaire et plusieurs brins extérieurs à ladite partie.

6. Lien selon la revendication 4 caractérisé en ce que l'enroulement du feuillard (1) dans la partie tubulaire de la chape (2) a une hauteur inférieure à celle-ci et permet le passage de l'extrémité libre du feuillard après enlèvement de l'aiguille pour réaliser un cerclage.

7. Matériel de pose d'un lien selon les revendications 1 à 6 caractérisé en ce qu'il est constitué d'une pince pourvue d'une rampe inclinée (13) sur lequel glisse le feuillard, ladite pince étant dotée d'un organe permettant d'exercer une traction controlée sur le feuillard, ladite traction étant exercée par rapprochement des branches (11-12) de la pince.

## Patentansprüche

1. Umschlingungsband für chirurgischen Einsatz bestehend aus einem flachen Stahlband (1), das an einem Ende mit der Unterseite (5) einer Klemme (3) verbunden ist, in der der freie Strang nach Umschlingung umgeschlagen und durch Befestigungsklappen blockiert wird. Es kennzeichnet sich dadurch, dass die Klemme aus einem röhrenförmigen Abschnitt mit quadratischem oder rechteckigen Durchschnitt besteht, der in bezug auf den freien Strang eine Unterseite (5) und eine Oberseite (6) aufweist, der freie Strang auf der Oberseite zur provisorischen Umschlingung umklappbar und die Oberseite (6) kürzer als die Unterseite (5) ist. Der freie Strang des Stahlbandes wird in der Klemme unterhalb der Oberseite (6) eingeführt und umgeschlagen. Durch Umklappen der Befestigungsklappen (3) wird eine definitive Blockierung des freien Strangs des Stahlbandes erreicht.

2. Umschlingungsband entsprechend Patentanspruch 1 gekennzeichnet dadurch, dass die Befestigungsklappen von der Oberseite der Klemme (6) durch eine Kerbe (7) in jeder Seitenwand der Klemme getrennt sind, die deren Umklappen auf eine geringere Höhe als die der Oberseite (6) ermöglichen.

3. Umschlingungsband entsprechend Patentanspruch 1 und Patentanspruch 2 gekennzeichnet dadurch, dass die Befestigungsklappen (3) am entgegengesetzten Ende des Stahlbandes (1) bei der Umschlingung eines Anatomieteils und am hinteren Teil der Klemme positioniert sind.

4. Umschlingungsband entsprechend Patentanspruch 1 und Patentanspruch 2 gekennzeichnet dadurch, dass das Stahlband (1) um die Unterseite der Klemme gerollt wird und aus mindestens einem äußeren und zwei inneren Strängen besteht.

5. Umschlingungsband entsprechend Patentanspruch 3 gekennzeichnet dadurch, dass dieses Umschlagen des Stahlbandes (1) mehrere innere Stränge im röhrenförmigen Abschnitt und mehrere Stränge in diesem Abschnitt darstellt.

6. Umschlingungsband entsprechend Patentanspruch 4 gekennzeichnet dadurch, dass dieses Umschlagen des Stahlbandes (1) im röhrenförmigen Abschnitt der Klemme (2) eine geringere Höhe als diese aufweist, wodurch nach dem Entfernen der Nadel das Einführen des freien Endes des Stahlbandes für die Ausführung der Umschlingung möglich ist.

7. Material für das Anbringen des Umschlingungsbandes entsprechend der Patenansprüche 1 bis 6, das aus einer Zange mit einer geneigten Zuführung (13) besteht, auf der das Band gleitet und einer Vorrichtung, die es ermöglicht eine kontrollierte Zugkraft auf das Band auszuüben. Diese Zugkraft wird durch Annähern der Zangengriffe (11-12) erreicht.

## Claims

1. Banding link for surgery including a flat strapping (1) forming one part with one end of the lower wall (5) of a coping (3) on which the free strand of the strapping is bent back after banding and locked in place by the fold-back flaps (3) characterised by the fact that the coping (3) has a tubular part with a square or rectangular cross-section with a lower wall (5) and an upper wall (6) in relation to the free strand in the coping, that the free strand can be bent back onto the upper wall (6) to make a temporary banding, that the upper wall (6) is shorter than the lower wall (5) and that the free strand of the strapping can be folded back into the coping (2) under the level of the upper wall (6) by bending back the flaps (3) to finally create an obstacle to block the strapping free strand.

2. Banding link as per claim 1 characterised by the fact that the flaps (3) are separated from the upper wall (6) of the coping by a groove (7) on each lateral wall of the coping enabling them to be bent back under the level of the upper wall (6).

3. Banding link as per claim 1 and claim 2 characterised by the fact that the flaps (3) are positioned opposite the development of the strapping (1) when it is rolled around the anatomic part to be banded and behind the coping.

4. Link as per claim 1 and claim 2 characterised by the fact that the strapping (1) is rolled around the lower wall (5) of the coping and shows at least one external strand and two internal strands.

5. Link as per claim 3 characterised by the fact that the winding of the strapping (1) shows several internal strands in the tubular part and several external strands on that part.

6. Link as per claim 4 characterised by the fact that the winding of the strapping (1) in the tubular part of the coping (2) has a height lower than the tubular part allowing the free end of the strapping to pass through once the needle has been removed to perform the banding.

7. Equipment to install a link as per claims 1 to 6 characterised by the fact that it comprises pliers provided with an inclined plane (13) on which the strapping slides, the said pliers being provided with a device to exert controlled traction on the strapping, the said traction being exerted by closing the arms (11-12) of the pliers.
